# EUROPEAN PATENT SPECIFICATION

(11) **EP 0 380 610 B1**
(45) Date of publication and mention of the grant of the patent: **29.03.1995**
(21) Application number: 89907444.7
(22) Date of filing: 19.05.1989
(51) Int. Cl.: C12N 5/00, C12M 3/04, C12M 1/40, C12N 1/12

(54) **BIOREACTOR DEVICE**
BIOREAKTOR
BIOREACTEUR

(30) Priority: 23.05.1988 US 197700; 18.05.1989 US 355115
(43) Date of publication of application: 08.08.1990
(73) Proprietor: REGENTS OF THE UNIVERSITY OF MINNESOTA, St. Paul, Minnesota 55401 (US)
(72) Inventor: HU, Wei-Shou, Falcon Heights, MN 55133 (US); SCHOLZ, Matthew T., Woodbury, MN 55125 (US)
(74) Representative: Patentanwälte Hauck, Graalfs, Wehnert, Döring, Siemons
(86) International application number: US8902228
(87) International publication number: WO8911529

(56) References cited:
- EP-A- 363 262
- EP-A- 0 155 237
- WO-A-89/00188
- FR-A- 565 349
- GB-A- 178 447
- US-A- 3 186 917
- US-A- 3 734 851
- US-A- 3 997 396
- US-A- 4 184 922
- US-A- 4 220 725
- US-A- 4 225 671
- US-A- 4 537 860
- US-A- 4 603 109
- US-A- 4 661 458
- US-A- 4 764 471

## Description

This application is a continuation-in-part of Application No. 197,700, filed on May 23, 1988, now abandoned.

### Field of the Invention.

This invention relates to an improved bioreactor apparatus for maintaining animal cells and genetically altered derivatives thereof in vitro for the continuous production of various cell products.

### Background of the Invention

Animal cells and genetically altered derivatives thereof are often cultivated in bioreactors for the continuous production of pharmaceutical proteins such as vaccines, monoclonal antibodies, and tissue type plasminogen activators. For example, pituitary cells can be cultured in vitro to produce growth hormones; kidney cells can be cultured to produce plasminogen activator; and cultured liver cells have been known to produce hepatitis A antigen. In these bioreactors, cells are essentially a system of catalysts and the medium supplies and removes the nutrients and growth inhibiting metabolites. To supply nutrients and remove metabolites, the medium in the bioreactor is changed either intermittently or continuously by fluid flow. However, because of their relatively small size and small density difference when compared to the medium, cells inevitably are withdrawn when the medium is changed, resulting in a relatively low cell concentration within the bioreactor. As a result of this low cell concentration, the concentration of the desired cell product is low in the harvested medium.

An ideal animal cell bioreactor would include three features: (1) cells would be retained in a viable state at high densities in the bioreactor apparatus as long as possible, with an almost infinite residence time; (2) high molecular weight compounds including expensive growth factors and the desired cell products, would have a long but finite residence time within the bioreactor to allow for both efficient nutrient utilization by the growing cells and also the accumulation of cell products to a high concentration; and (3) the low molecular weight compounds, including less expensive nutrients and inhibitory substances, should have a very short residence time within the bioreactor to reduce inhibition of cell product formation.

Numerous procedures and devices for in vitro cell culture production of biomolecules have attempted to achieve these goals in the past. In relatively simple systems, the cells have been grown in tissue flasks and roller bottles in the presence of a suitable nutrient media. More complex systems have used capillary hollow fiber membranes as a surface support for the cells in conjunction with a means for supplying nutrient media to the cells.

For example, U.S. Patent No. 4,537,860 to Tolbert describes a static cell culture maintenance system for maintaining animal cells in a substantially arrested state of proliferation with continuous secretion of cell product. The cells are retained within a reactor vessel chamber in a semi-rigid matrix having interstices for passage of fluid nutrient medium. Fresh nutrient medium is supplied by perfusion into the matrix through relatively low porosity tubes which are suspended in the reactor chamber and which substantially traverse the matrix. High porosity tubes are available to withdraw expended medium and cell product.

A membrane-type cell reactor is also shown in Construction of a Large Scale Membrane Reactor System with Different Compartments for Cells, Medium and Product, Develop. Biol. Standard., Vol. 66, pages 221-226 (1987). In this membrane system, cells are immobilized in a wire matrix where different membranes separate the cells from the medium and the cells from the cell product. The membrane lying between the medium and the cells is an ultrafilter with a useful molecular weight cut-off preventing the particular cell product from crossing into the medium compartment. The other membrane is a microfiltration membrane which separates the cells from a cell product chamber. With this configuration it is possible to feed the cells continuously and harvest the collected cell product at a distinct time interval without removing cells.

While these reactor systems attempt to tackle the problems of maintaining a high cell concentration to consequently harvest a high level of cell product, there is much room for improvement. Accordingly, the bioreactor of the present invention provides an in vitro cell culture system which maintains a large number of cells for an almost infinite residence time with continuous or intermittent cell product secretion.

### Summary of the Invention

In accordance with the bioreactor of the present invention, animal cells are maintained in vitro over a sustained period of time. Briefly, this bioreactor apparatus consists of two chambers, a feed and waste chamber and a cell chamber, separated by a selectively permeable ultrafiltration membrane. This membrane selectively allows nutrients and cell waste products to cross between the chambers but not the desired cell product. Within the cell chamber, a biocompatible, three-dimensional matrix entraps the animal cells. Due to the presence of this biocompatible matrix, the cell chamber generally has a gel phase, i.e., the biocompatible matrix, and a liquid phase containing a concentrated solution of the cell product to be harvested. Thus, the bioreactor of the present invention uses only two chambers to achieve three distinct zones within the bioreactor apparatus. Spent nutrients and cell waste products are withdrawn through an outlet means in flow communication with the feed and waste chamber. A withdrawal means, in flow communication with the cell chamber, may also be provided for collection of the desired cell product without disturbing the producing cells.

Various features and advantages that result from a bioreactor apparatus using the principles of this invention are pointed out with particularity in the claims. However, reference should also be made to the drawings and the accompanying detailed description of the invention for a more thorough understanding of the invention.

### Brief Description of the Drawings

Figure 1 is an abstract representation of a bioreactor apparatus that uses the inventive principles of the present invention;
Figure 2 is a pictorial representation of an embodiment of a bioreactor apparatus that uses the inventive principles of this disclosure;
Figure 3 is a schematic view of a system using a flat bed type bioreactor apparatus;
Figure 4 is a schematic view of a system using a hollow fiber bioreactor apparatus;
Figure 5 is an exploded isometric view of an embodiment of the present invention;
Figure 6 is a plan view of a base plate used in the embodiment depicted in Figure 5;
Figure 7 is a plan view of a membrane used in the embodiment depicted in Figure 5;
Figure 8 is a plan view of a media plate used in the embodiment of the invention depicted in Figure 5;
Figure 9 is a plan view of a cell product plate used in the embodiment depicted in Figure 5;

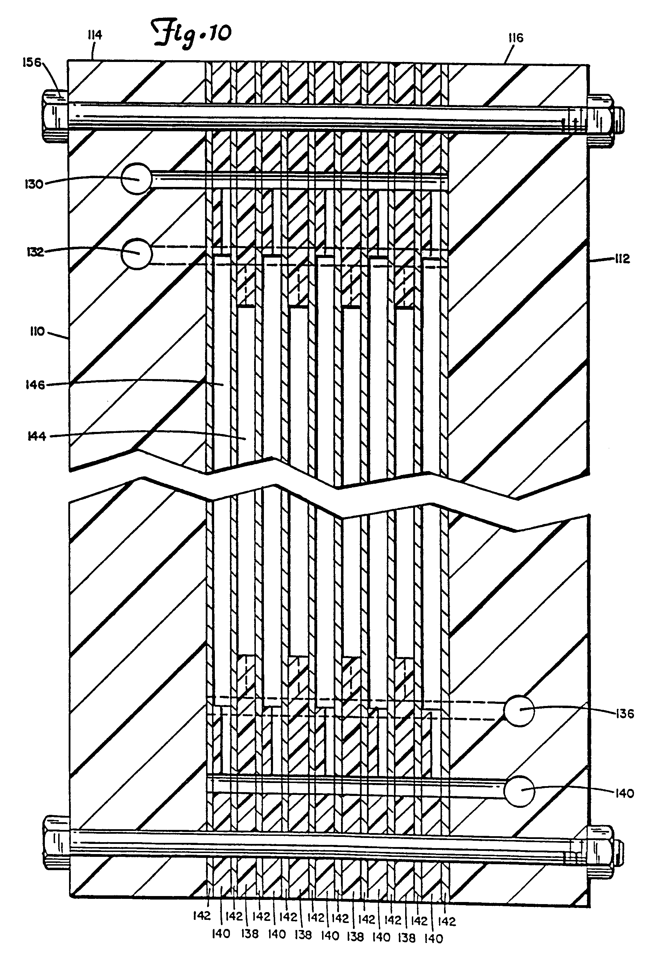

Figure 10 is a cross-sectional view of the embodiment depicted in Figure 5 through line 10-10;
Figure 11 is a side cross-sectional view of another embodiment of the present invention;
Figure 12 is a cross-sectional view of the embodiment depicted in Figure 11 through line 12-12;
Figure 13 is a graph;
Figure 14 is a graph;
Figure 15 is a graph;
Figure 16 is a graph;
Figure 17 is a graph;
Figure 18 is a graph;
Figure 19 is a graph;
Figure 20 is a graph;

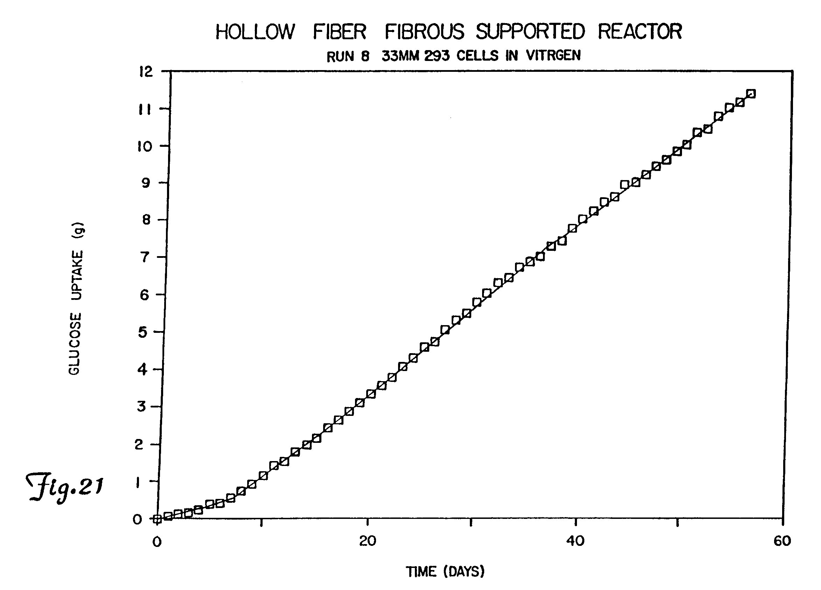

Figure 21 is a graph;
Figure 22 is an abstract representation of a bioreactor apparatus that uses the inventive principles of the present invention; and
Figure 23 is a cross-sectional view of the embodiment depicted in Figure 22.

### Detailed Description of the Invention

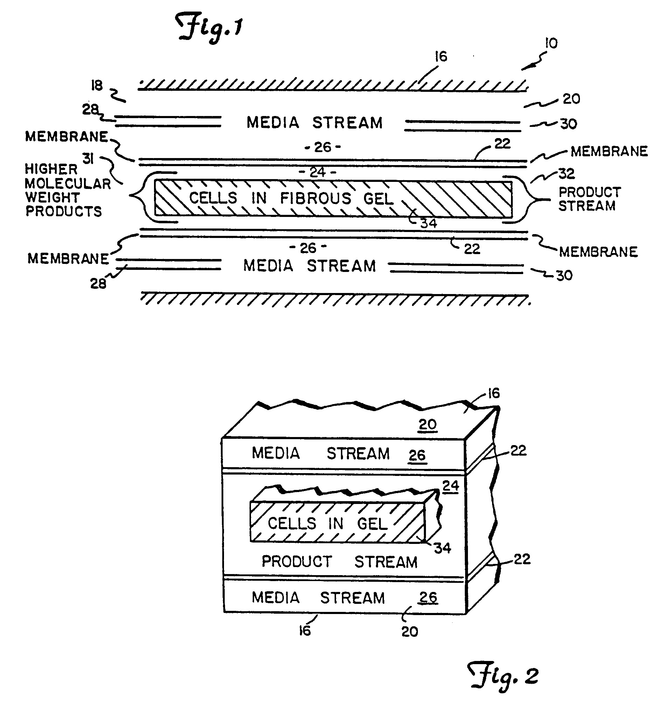

With reference to the ideas depicted in Figures 1 and 2, a bioreactor 10 according to the inventive principles of this disclosure would generally include two chambers within a housing means 16 having a proximal end 18 and distal end 20. A selectively permeable membrane 22 lies within housing means 16. Membrane 22 extends from proximal end 18 to distal end 20 to divide the interior of housing means 16 into a cell chamber 24 and a feed and waste chamber 26.

The preferred membrane selectively allows low molecular weight compounds, such as nutrients and cell waste products, to cross between cell chamber 24 and feed chamber 26. However, membrane 22 does not allow high molecular weight compounds, such as the cell product to be harvested, to cross between the two chambers. The membrane must be permeable to essential nutrients and toxic waste products but must also retain the desired cell products in the cell chamber. Naturally, the desired upper molecular weight limit of the membrane will be chosen such that it is smaller than the molecular weight of the desired cell product. Thus, a suitable membrane for a cell product having a molecular weight exceeding 14,000 would be constructed of processed cellulose having an upper molecular weight limit generally ranging from 12,000 to 14,000. Such a membrane is commercially available from Spectrum Medical Industries, Inc. of Los Angeles, California, under the trade name Spectra/Por 4. Other ultrafiltration membranes that could be used with a bioreactor system of the present invention include polysulfone, nylon, polypropylene, polyester/polycarbonate, teflon, ionically charged membranes, cellophane, nitrocellulose polyethylene and ceramics. A few commercial examples include polycarbonate and polyester Nucleopore® membrane filters from Nucleopore Corporation in Pleasanton, California; polysulfone PTGC membranes from Millipore of Bedford, Massachusetts; and nitrocellulose Collodion® membrane filters from Schleicher and Schnell, Inc. in Keene, New Hampshire.

Feed and waste chamber 26 supplies the cells with nutrient medium and carries away expended medium and cell waste products that have crossed membrane 22 to chamber 26. Inlet means 28 in flow communication with feed and waste chamber 26 are provided for supplying the desired nutrient medium. Outlet means 30 further communicates with feed and waste chamber 26 to remove expended medium and cell waste products.

Growth chamber 24 consists of two distinct phases: a substantially insoluble, biocompatible matrix 34 entrapping animal cells to form a gel phase; and a concentrated solution of the secreted cell product forming a liquid phase. The term insoluble as used herein refers to a composition which is capable of being separated from the cell culture medium by filtration. This bisectional cell chamber 24 is formed when a suitable matrix precursor/cell suspension is placed within growth chamber 24. The cell containing matrix precursor suspension contracts within cell chamber 24 to form a generally dense, insoluble, cell-biocompatible matrix 34. Utilizing biocompatible matrix 34, cells can be maintained in vitro for a very long period of time. Residence times of up to 90 days have been reached.

Generally, the cell-biocompatible matrix is formed when the chosen cells are mixed with a matrix precursor solution at lower temperatures (e.g., 0°C. to 30°C.), at lower pH values (e.g., 2 to 5.5), at both a lower temperature and a lower pH value, or in a solution of different ionic makeup. The chosen matrix precursor is preferably initially in a soluble form to create this suspension. The cell-matrix precursor suspension is then introduced into the cell chamber 24 through inlet means 31. When the pH, the temperature, or ionic character is changed from the initial value, polymerization or aggregation occurs with the resulting polymer chains forming insoluble aggregates (e.g., pH value increased to the range of 6.8 to 7.4, temperature increased to the range of 37°C. to 45°C.). These insoluble aggregates will further aggregate to form fibers. These fibers, in turn, entrap the cells creating what is referred to as the substantially insoluble, cell-biocompatible matrix 34.

It is further desired that the chosen matrix precursor have the ability to rapidly form a substantially insoluble, biocompatible matrix in situ to uniformly entrap the cells, before the cells settle. The chosen matrix precursor should preferably form the fibrous matrix upon a physical or chemical change in the cell-matrix precursor suspension. Such a change could be the result of a shift in pH or temperature value, or both, addition of a comonomer or any other initiator of polymerization, or any combination of these methods. Depending on the chosen matrix precursor, the formed matrix could be the result of polymerization, aggregation, ionic complexation or the like.

For the sake of convenience, it should be understood that wherever the term polymer or aggregate is used to refer to the matrix construction, the matrix is not limited to compounds with those characteristics. Any biocompatible, substantially insoluble matrix that forms in situ and entraps cells, at least initially, is considered to be within the scope of the present invention. Likewise, the matrix precursor should be read to include, but not be limited to, all compounds which tend to polymerize or aggregate or the like to form the matrix in situ.

Due to contraction either caused by the cells or the matrix itself, the cell-biocompatible matrix will, in some cases, but not all, contract to one quarter of the original volume occupied by the mixture in a few hours or days. For the present invention it is not necessary for the cell-biocompatible matrix to contract to this extent. A cell-matrix which contracts to approximately 90% of the original volume occupied by the mixture is desired. A cell-matrix which has contracted to approximately 75% of the original volume occupied is even better. A cell-matrix which has contracted to approximately 50% of the original volume is even more preferred. However, the most desirable cell-matrix will contract to approximately one-third of the original volume occupied by the mixture.

After contraction has occurred, cell chamber 24 has two distinct zones, the cell-biocompatible matrix zone and a liquid zone in which high molecular weight compounds produced by the cells will accumulate. Cell products can be harvested periodically or continuously through withdrawal means 32.

The resulting matrix must be at least partially insoluble in the cell media that is employed under optimum culture conditions, i.e., pH = 7.0-7.4; temperature = 37°C; and osmolarity = 275 - 400 milliosmoles. In addition the cell-biocompatible matrix must be non-cytotoxic and sterilizable. Numerous matrix precursor compounds can be used to create the desired cell-biocompatible matrix.

One compound that has been found to form a particularly suitable matrix is collagen. Sterile, high purity native ateleopeptide collagen Type I is commercially available from Collagen Corporation in Palo Alto, California under the trade name Vitrogen™ 100. Teleopeptide collagen Type I has also proven to be useful and is available in a relatively pure form from Gottefosse Corporation located in Elmsford, New York under the trade name Pancogene S™. Whenever the term collagen is used in this description, it should be read to include any type of collagen or modified collagen which is at least partially insoluble under optimum cell culture conditions. For example, collagen may be modified according to the techniques of United States Patent No. 4,559,304 to Kasai, et al., the disclosure of which is incorporated by reference herein.

A collagen-chitosan mixture may also be used. Suitable chitosan, a derivative of chitin in which many of the N-acetyl linkages have been hydrolysed to leave the free amine, can be obtained from Protan Labs of Redmond, Washington in a dry state under the label Ultrapure Chitosan. As in the case of collagen, it should be recognized that the chitosan can also be chemically modified and still be an effective means for forming the matrix. In addition, the in situ polymerization of a fibrinogen and thrombin mixture to form fibrin has been successfully employed.

Other materials which would meet the requirements of this system include: (1) polyamines wherein the subunits which make up the polymer have a pkₐ value generally ranging from 7 to 10, such as collagen and chitosan. Such polyamines are soluble in a cell culture media at pH values generally in the range of 2 to 5.5 when in a protonated form and partially insoluble in a cell culture media at pH values generally ranging from 6.8 to 7.4 when in a partially unprotonated form; (2) a mixture of water soluble polyanionic polymers and polycationic polymers. This mixture would associate through ionic bonds and fall out of solution; and (3) polymers, such as cellulose ethers, which are soluble in a cell culture media at temperatures ranging from 0°C. to 30°C. but insoluble in a cell culture media at higher temperatures, such as those generally ranging from 32°C. to 45°C. have also been contemplated.

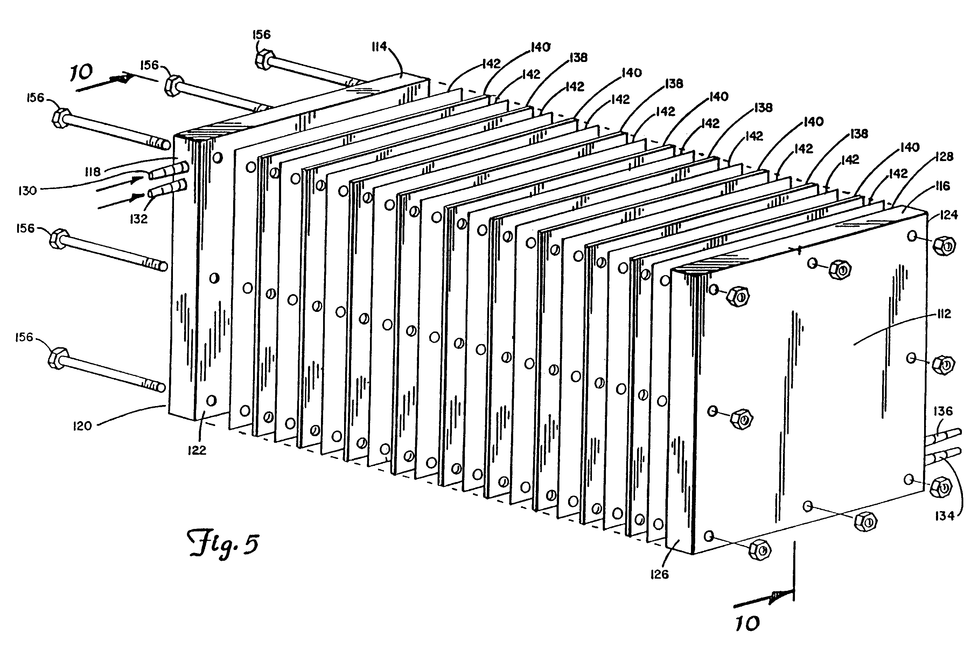

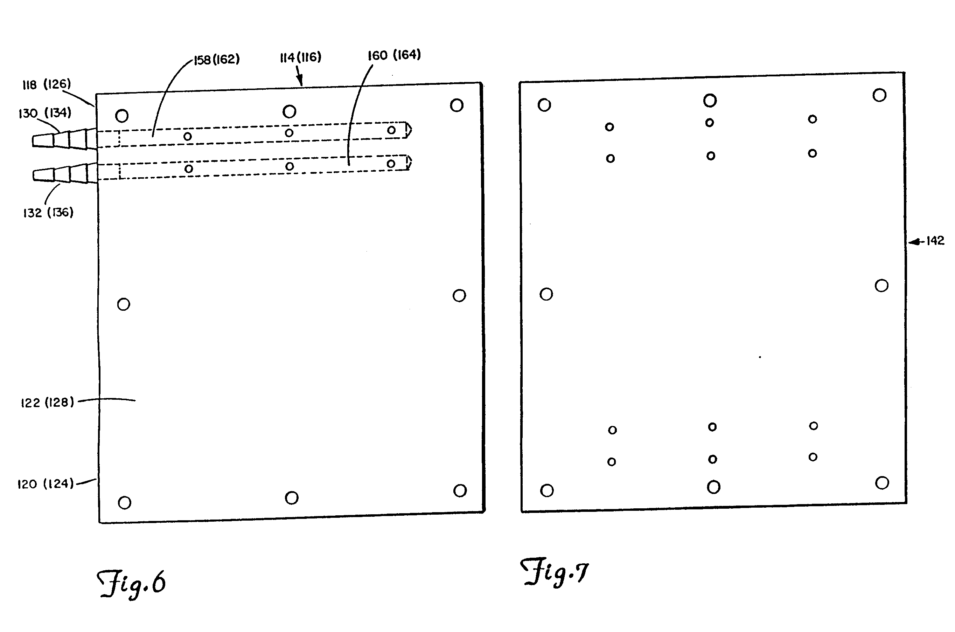

These principles were incorporated in a flat bed type embodiment 100 of the present invention as shown in Figures 5 through 9. The external housing of flat bed bioreactor 100 is formed by exterior faces 110 and 112 of a first base plate 114 and a second base plate 116. Figure 6 shows base plate 114 and 116 in more detail. Base plates 114, 116 are preferably made of polycarbonate because it is transparent and steam sterilizable. However, base plates 114 and 116 could be constructed of any suitable plastic or metal. First base plate 114 has proximal 118 and distal 120 ends and exterior 110 and interior faces 122. Second base plate 116 has proximal 124 and distal 126 ends and exterior 112 and interior faces 128. First base plate 114 has first 130 and second 132 fluid inlet means.

Both fluid inlet means 130, 132 are preferably located near the proximal end 118 of first base plate 114, with second fluid inlet means 132 located slightly posterior to or below first fluid inlet means 130. Second base plate 116 has first 134 and second fluid outlet means 136. Both fluid outlet means are located near distal end 126 of second base plate 116, preferably with second fluid outlet means 136 slightly anterior to or above first fluid outlet means 134. Second fluid outlet means 136 and second fluid inlet means 132 may be capped with a rubber septum or equipped with a short piece of tubing terminated in a valve if only periodic harvesting of cell product is desired.

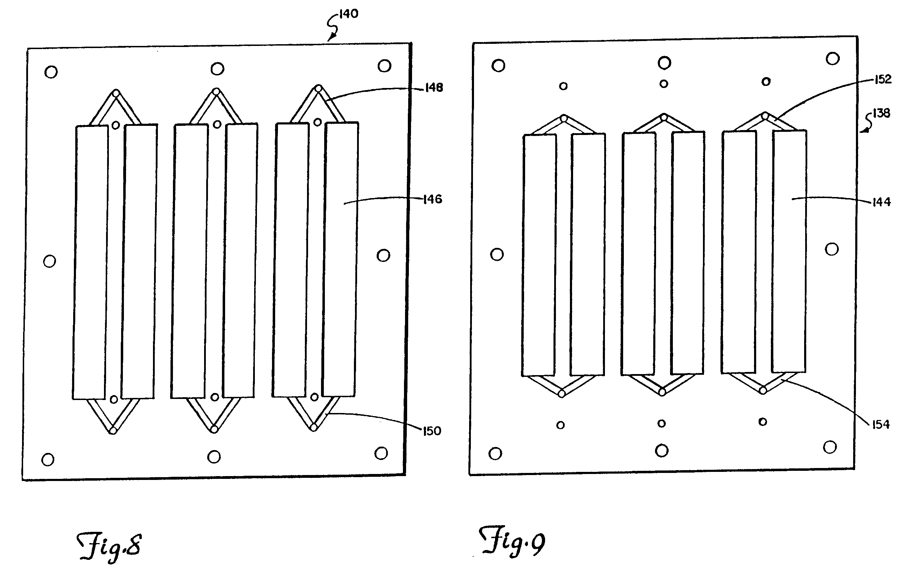

Between interior faces 122, 118 of first base plate 114 and second base plates 116 are alternating cell growth plate(s) 138, selectively permeable membranes 142 and nutrient medium plate(s) 140. Bioreactor 100 has at least one cell growth media plate 138 as shown more particularly in Figure 9. Each cell growth plate 138 has at least one longitudinal window 144. The length of the cell growth plate window(s) 144 is substantially equal to the distance from second fluid inlet means 132 to second fluid outlet means 136 as measured in the assembled flat bed bioreactor 100.

As shown more particularly in Figure 8, bioreactor 100 also has at least one nutrient medium plate 140. Each nutrient medium plate 140 has at least one longitudinal window 146. The length of nutrient medium plate window(s) 146 is substantially equal to the distance from first fluid inlet means 130 to first fluid outlet means 134 as measured in the assembled flat bed bioreactor 100. Thus, the length of nutrient medium plate window(s) 146 is slightly longer than the length of cell plate window(s) 144. Naturally, the length of longitudinal windows 144, 146 depends upon the location of first and second fluid inlet and outlet means 134, 136. Thus, it is possible that window 144 may be slightly longer than the length of nutrient medium window(s) 146. In this case the length of the nutrient medium window(s) 144 will be substantially equal to the distance from the first fluid inlet means 130 to the first fluid outlet means 134, as measured in the assembled flat bed bioreactor 100, and the length of the cell plate window(s) 146 will be substantially equal to the distance from the second fluid inlet means 132 to the second fluid outlet means 136.

In the preferred embodiment, at least one first medium channel(s) 148 is in flow communication with first fluid inlet means 130 and nutrient medium plate window(s) 146. At least one second medium channel(s) 150 is in flow communication with nutrient medium plate window(s) 146 and first fluid outlet means 136. At least one first cell channel(s) 152 is in flow communication with second fluid inlet means 132 and cell growth plate window(s) 144. At least one second cell channel(s) 154 is in flow communication with cell growth plate window(s) 144 and second fluid outlet means 136. Channels 148, 150, 152, and 154 do not extend through their respective plate 138, 140. On first base plate 114, in flow communication with first and second fluid inlet means 130, 132 and channels 148, 152 are preferably first and second fluid inlet flow manifolds 158, 160. Likewise, first and second fluid outlet flow manifolds 162, 164 on second base plate 116 are in flow communication with first and second fluid outlet means 134, 136 and channels 150, 154. Preferably, the bore size of manifolds 160, 164 are small to avoid dilution of the product stream as the product is withdrawn.

Selectively permeable membranes 142 as shown in Figure 7 and used in flat bed bioreactor 100 of this invention are pervious to the passage of nutrients and cell waste products from one side of membrane 142 to the other, while being substantially impervious to the passage of the animal cells and desired cell products from one side of membrane 142 to the other.

Base plates 114, 116, plates 138, 140 and membranes 142 are preferably assembled together in the following sandwich-type fashion to form flat plate bioreactor 100 of the present invention. Exterior faces 110, 112 of first 114 and second base plates 116 are positioned with each face outward from each other, forming the exterior housing of bioreactor 100. Plates 138, 140 and membranes 142 are sandwiched between base plates 114, 116, so that nutrient medium plate(s) 140 alternates with cell growth plate(s) 138, while each membrane 142 separates each plate from each other plate and from interior face 122, 128 of each base plate 114, 116. Securing means 156, such as bolts, screws, clamps or the like can be used to hold the assembled sandwich bioreactor apparatus.

It is not necessary for operation of bioreactor 100 that membrane 142 be placed between base plates 114, 116 and medium plate(s) 138, 140. However, when used in this fashion, the membranes serve as an effective gasket. In forming the sandwich structure of bioreactor 100 the first and last plate of the sandwich, excluding base plates 114, 116, is preferably a nutrient medium plate 140. Preferably, a flat bed bioreactor 100 of this invention is formed with a plurality of cell growth plates 138 and nutrient medium plates 140 and a plurality of membranes 142.

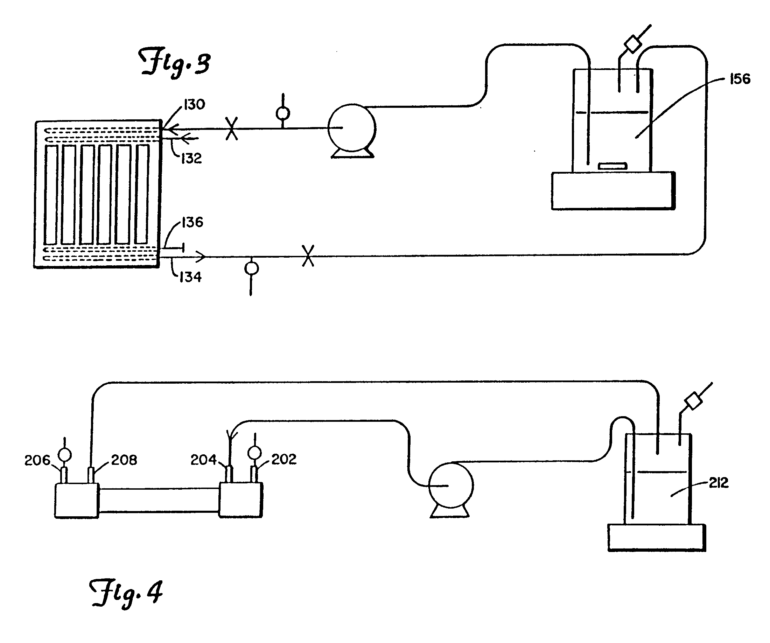

In operation, the chosen cell nutrient media is pumped with a peristalic pump, as shown in Figure 3, from a media reservoir 156 through first fluid inlet means 130 and first medium channel(s) 148 to nutrient medium plate window(s) 146. A suitable pump is a variable speed Masterflex Cat. No. 7533-30 with size 16 Masterflex silicone tubing from Cole Palmer in Chicago, Illinois. Medium continues through nutrient medium plate window(s) 146 to second medium channel(s) 150 and subsequently out of flat bed bioreactor 100 through first fluid outlet means 134. The cell-matrix precursor suspension is introduced through second fluid inlet means 132, through first cell channel(s) 152 into cell growth plate window(s) 144. Second fluid outlet means 136 is preferably capped.

After the cell-matrix precursor suspension is introduced into cell growth plate window(s) 144, the cell entrapping, substantially insoluble matrix 34 is formed in situ. Cells are maintained by the continuous flow of nutrient medium which crosses membrane 142. Toxic cell waste products diffuse across membrane 142 to nutrient medium plate window(s) 146, where they are carried out of bioreactor 100. Due to their high molecular weights, cell products to be harvested do not cross membrane 142.

To periodically harvest the cell products, a syringe, or other withdrawing means may be inserted into second fluid outlet means 136. For continuous harvesting, either a pump or a sample flow control valve may be employed. Alternatively, fluid may be introduced into the cell chamber, displacing the cell products to be harvested.

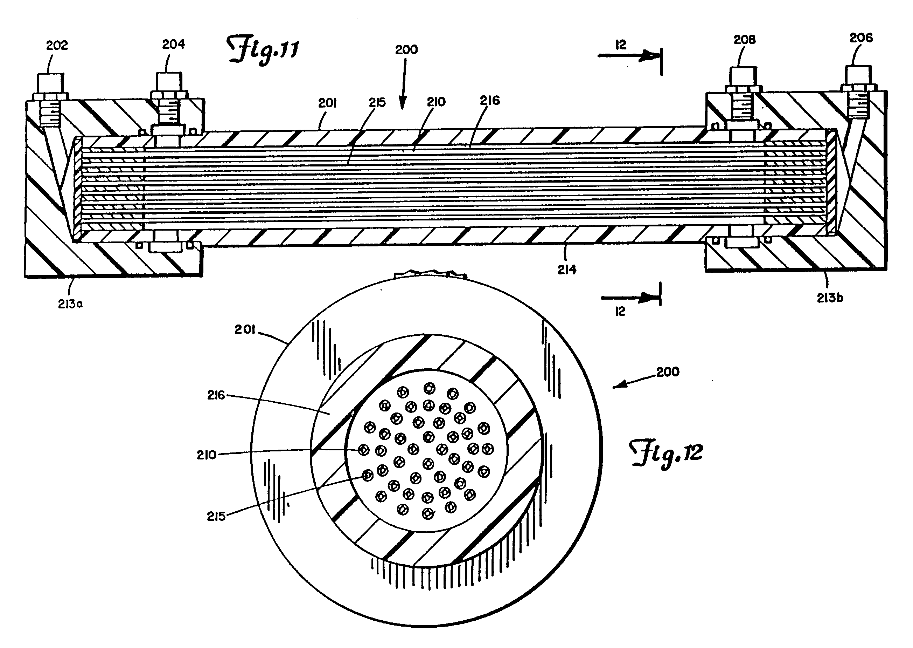

Alternatively, the principles of the present invention can be employed in a hollow fiber bioreactor 200 as shown in Figures 11 and 12. A suitable hollow fiber assembly is the Amicon PN 5407 Model DH4 from Amicon, a division of W.R. Grace & Co. in Danvers, MA, with the pressure control value and filter frits removed. An Amicon HlP30-43 hollow fiber membrane assembly was used for this example with an upper molecular weight limit of approximately 30,000. The hollow fibers of this assembly were formed of polysulfone, although any suitable membrane composition as discussed above may also be successfully employed.

A suitable hollow-fiber assembly 200 has a housing 201 having spaced end portions 213a, 213b defining a chamber 214 therebetween. Housing 201 has a first 202 and second 204 fluid inlet means with second fluid inlet means 204 positioned generally toward the inside of first fluid inlet means 202. Housing 201 also has a first 206 and second 208 fluid outlet means, with second fluid outlet means 208 positioned generally toward the inside of first fluid outlet means 206. While housing 201 is depicted in Figures 11 and 12 as being cylindrical, its shape is not so limited. Any housing may be successfully employed which will house hollow fibers.

Within housing 201 is at least one selectively permeable hollow fiber 210, pervious to the passage of nutritents and toxic cell waste products while substantially impervious to the passage of cells and the desired cell product, extending the length of housing 201. Hollow fibers 210 divides chamber 214 into an intracapillary space 215 within hollow fiber 210 and an extracapillary space 216 outside hollow fiber 210. Intracapillary space 215 and extracapillary space 216 communicate only through the walls of hollow fiber 210. Preferably, intracapillary space 215 provides a cell chamber for cells entrapped in the chosen matrix while extracapillary space 216 provides a nutrient medium, or feed and waste chamber. These roles may be reversed, if desired. Preferably, a plurality of fibers would be employed. The interior lumens of hollow fibers 210 are in flow communication with first fluid inlet means 202 and first fluid outlet means 206. Extracapillary space 216 is in flow communication with second fluid inlet means 204 and second fluid outlet means 208.

In operation, as shown in Figure 4, nutrient medium would be pumped from reservoir 212 through second fluid inlet means 204, if extracapillary space 216 is to be used as the nutrient medium or feed and waste chamber. The medium travels through extracapillary space 216 and exits housing 201 through second fluid outlet means 208. The matrix precursor-cell suspension is introduced into hollow fibers 210 through first fluid inlet means 202, if intracapillary space 215 is to be used as the cell chamber. First fluid outlet means 206 is capped with a rubber septum or a short piece of tubing terminated in a valve. The substantially insoluble, cell-matrix subsequently forms in situ within hollow fibers 210.

Nutrient medium crosses the fibrous membrane wall of hollow fiber 210 to feed the entrapped cells. Cell waste products and expended medium perfuse through the walls of hollow fibers 210 into the extracapillary space where they are carried away with the medium stream. The desired cell product can be harvested continuously or periodically through first fluid outlet means 206.

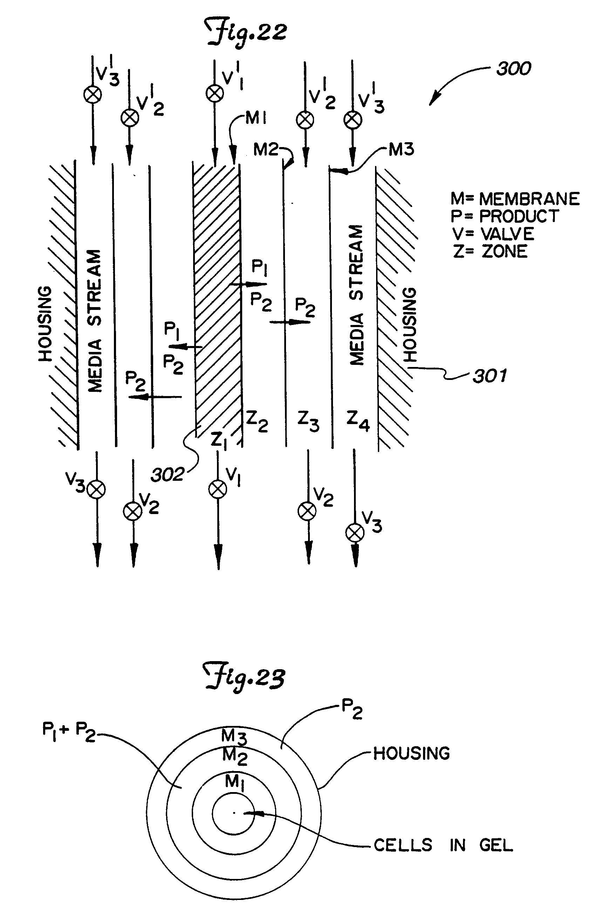

A multizone bioreactor design could also be employ the principles of the present invention. This bioreactor configuration would be particularly useful where harvesting of more than one cell product is desired. In this configuration, the cell products to be harvested, P₁ and P₂, would have significantly different molecular weights. For example, cell product P₁ would have a molecular weight that is significantly greater than that of cell product P₂. As shown in Figures 22 and 23, a multizone bioreactor according to the principles of the present invention, generally referred to as 300, would consist of multiple concentric, selectively permeable hollow fibers M₁, M₂, and M₃ of different pore sizes sealed in a housing 301 having spaced end portions and defining a chamber therebetween.

As depicted in Figure 22, within the housing chamber of multizone bioreactor 300 would be a first selectively permeable hollow fiber M₁, which would be preferably previous to the passage of nutrients, toxic cell waste products and cell products P₁ and P₂, while substantially imprevious to the passage of cells. Within the intracapillary space of first hollow fiber M₁ is a first zone Z₁. A second selectively permeable hollow fiber M₂ would be concentric to said first hollow fiber M₁. Second hollow fiber M₂ would preferably be substantially pervious to the passage of nutrients and cell waste products while impervious to at least one cell product e.g. P₁. Second hollow fiber M₂ creates a second zone Z₂ within the intracapillary space intermediate first hollow fiber M₁ and second hollow fiber M₂.

A third selectively permeable hollow fiber M₃ would be concentric to second hollow fiber M₂. Third hollow fiber M₃ would preferably be substantially pervious to the passage of nutrients and cell waste products while impervious to the passage of all desired cell products, here, P₁ and P₂. Third hollow fiber M₃ creates two additional zones: a third zone Z₃ is created in the intracapillary space intermediate second hollow fiber M₂ and third hollow fiber M₃ while a fourth zone Z₄ is created within the extracapillary space intermediate third hollow fiber M₃ and housing 301.

With this configuration, first hollow fiber M₁, second hollow fiber M₂ and third hollow fiber M₃ would allow nutrients and cell waste products to cross from zone Z₁ to zone Z₄ and from zone Z₄ to zone Z₁. However, cell product P₁ would be contained within zone Z₂. Cell product P₂, on the other hand, would be able to freely diffuse through second hollow fiber M₂ into zone Z₃. The pore size of third hollow fiber M₃, however, would prevent cell product P₂ from diffusing into zone Z₄. It should be understood, however, that greater than or less than four zones may be possible, depending upon the number of cell products to be harvested and the desired concentration. The embodiment shown in Figures 22 and 23 is not intended to be a definitive representation of a multizone bioreactor.

A suitable, commercially available concentric hollow fiber bioreactor for use with the present invention is available from Setec, Inc. of Livermore, California under the trademark TRICENTRIC®. The hollow fibers of this assembly are formed of polypropylene, although any suitable membrane composition discussed above may also be successfully employed.

In operation, a suitable matrix precursor/cell solution would be introduced into zone Z₁ through valve means V₁', which would be in flow communication with zone Z₁, where the suspension subsequently contracts to form a generally dense, insoluble cell - biocompatible matrix 302. Matrix 302, and cell products, can be removed through valve means V₁ which is also in flow communication with zone Z₁. With matrix 302, cells can be maintained in vitro for a very long period of time.

Nutrient media is passed by means of valve means V₃ and V₃' through zone Z₄. Valve means V₃ and V₃' are in flow communication with zone Z₄. The low molecular weight nutrients freely diffuse through hollow fibers M₁, M₂ and M₃ to maintain the cells residing in zone Z₁. Similarly, low molecular weight cell waste products and inhibitory metabolites are able to diffuse through the series of concentric hollow fibers into zone Z₄. The media stream in zone Z₄ carries away expended nutrient medium and cell waste products from the assembly.

The residence times of cell products P₁ and P₂ are controlled by the operator. These products can be harvested either continuously or intermittently through valve means in flow communication with the desired zone. As depicted in Figure 22, the cell product stream from zone Z₂ would contain both cell products P₁ and P₂ whereas that of zone Z₃ would contain only cell product P₂. Cell product P₂ could be readily removed from zone Z₃ through use of valve means V₂ and V₂'.

If a relatively pure stream of P₁ was desired, on the other hand, valve means V₁', V₂ and V₂' could be opened and valve means V₁, V₃ and V₃' closed while nutrient medium is pumped into zone Z₁ through valve means V₁. In this manner, nutrient medium would be forced to diffuse through second hollow fiber M₂, carrying residual cell product P₂ with it, and out of the assembly through valve means V₂. Since cell product P₁ cannot pass through hollow fiber M₂, in this configuration, cell product P₁ would remain in zone Z₂ and could be subsequently harvested. This method would result in some dilution of cell product P₂ but the stream of cell product P₂ would still be several times more concentrated than if the cells were grown in conventional bioreactor systems.

Other designs may also be employed. The essential design feature of a bioreactor apparatus of the present invention is the use of two chambers to achieve at least three distinct zones within the bioreactor by incorporating an in situ forming matrix.

A bioreactor apparatus using the principles of the present invention provides high oxygen transfer to the entrapped cells to maintain cell viability within the bioreactor with a low shear flow. Moreover, because of the concentrated cell product that is withdrawn, cell product recovery costs are reduced. Indeed, in many cases a substantially cell free cell product is achieved. A bioreactor apparatus according to the principles of the present invention may also be used to harvest nonsurface dependent cells such as AFP-27. These cells eventually slough off the matrix due to cell multiplication and can be harvested along with the desired cell product.

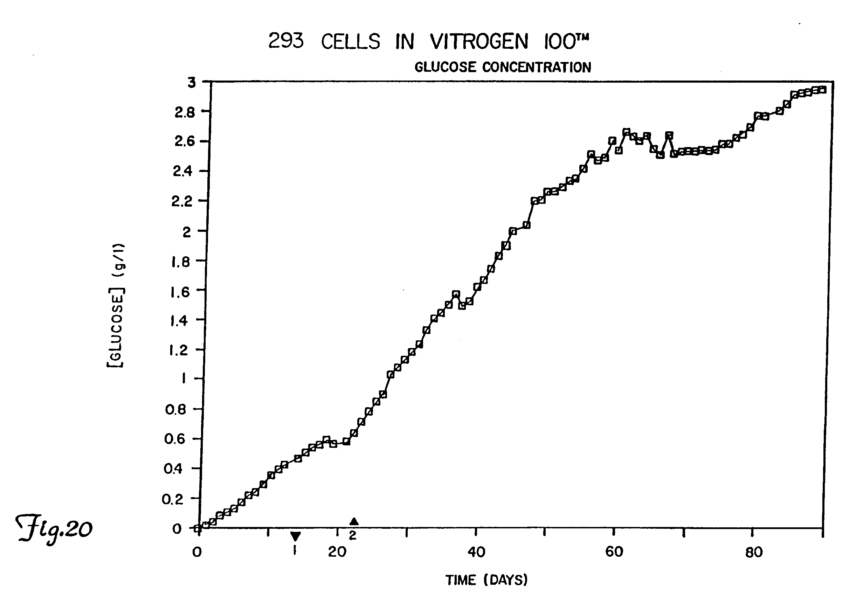

Our results further demonstrate that rapid start-up of this bioreactor apparatus is possible as well as step changes from serum containing medium to serum free medium and in many cases even protein free medium as shown in Example 6. A "step change" means to change instantaneously rather than gradually. In the context of this application, step change refers to the removal of medium containing serum entirely and the subsequent replacement with serum free medium. As shown by the triangles in Figure 20, after serum free medium is introduced into the bioreactor in a step change fashion, rather than a gradual or prolonged transition period, the cells remain viable. Triangle 2 indicates that time when serum free medium was introduced into the system. The rapid change to a serum free medium did not result in a decreased glucose consumption rate or cell death as usually occurs in other devices. By allowing for the rapid introduction of serum free medium, the bioreactor apparatus of the present invention can be set up and operated quickly and efficiently.

The following examples will more fully illustrate how animal cells and their genetically altered derivatives can be cast into a substantially insoluble biocompatible matrix. The resulting cellular response in these systems is also described.

### EXAMPLE 1: 293 Cells in a Collagen Matrix

In a laminar flow HEPA filtered hood, two sterile 15 ml. screw-cap tubes, Tube A and Tube B, were prepared for use. To tube A, 1.75 ml. of modified Dulbecco's Modification of Eagles medium (DME) was added. This medium had previously been prepared to twice the normal concentration and which included 10% fetal bovine serum (FBS); 300 »g/ml geneticen, 200 »g/ml hygromycin B, and 2»g/ml Vitamin K. The resulting medium mixture was sterilized by filtration. 0.10 ml. of steam sterilized 0.1N NaOH was added to Tube A. 1.0 ml of sterile VITROGEN 100™ was added to Tube B. Both tubes were sealed and placed in an ice water bath to cool the solutions to generally less than 4°C.

Genetically engineered human kidney epithelial cells ("293 cells") were used for this example. The base cells are publicly available under Deposit No. CRL 1573 at the ATCC in Rockville, Maryland. Using standard and well known techniques, these cells can be genetically manipulated so that the cells produce Protein C, a natural anticoagulant protein. See e.g., Lawrence H. Clouse, and Philip C. Comp., The Regulation of Hemostasis: The Protein C System, 314(20) The New England Journal of Medicine 1298 (May 15, 1986); P.C Comp, and L.H. Clouse, Plasma Proteins C and S: The Function and Assay of Two Natural Anticoagulants, Laboratory Management, pp. 29-32 (December 1985).

The 293 cells were grown to confluence in a 75 cm² tissue culture flask in a solution of DME, which included 5% FBS; 300 »g/ml geneticin; 200 »g/ml hygromycin B; and 2 »g/ml vitamin K ("DME + Ab solution") according to standard tissue culture techniques. See e.g., R. Ian Freshney, Alan R. Liss, Culture of Animal Cells, A Manual of Basic Technique, (1983). Using aseptic techniques, the medium was removed from the flask and the cells were gently washed with 5.0 ml. of phosphate buffered saline (PBS) solution to remove residual serum. The PBS solution contained 8 g/l sodium chloride, 0.2 g/l potassium chloride, 2.0 g/l sodium phosphate dibasic and 0.40 g/l potassium phosphate monobasic. The PBS solution was then removed.

1.0 ml. of a 0.25% Trypsin solution in PBS was subsequently added. The cells and solution were incubated for 5 minutes at 37°C. After the incubation period, a solution of DME + Ab was added to inactivate the trypsin. Cells were sloughed off the surface and suspended in the added medium.

Again using aseptic techniques, the contents of Tube A were added to the contents of Tube B. Immediately following this addition step, 0.9 ml. of the cell suspension (6.15 x 10⁷ total cells) was added to Tube B. The contents of Tube B were then mixed well by inverting the tube several times. The resulting mixture was poured into a 34 mm tissue culture dish and incubated at 37°C to form a substantially insoluble cell-biocompatible matrix. The amount of matrix contraction was measured daily using the methods described in Bell et al, "Production of a Tissue-Like Structure by Contraction of Collagen Lattices by Human Fibroblasts of Different Proliferative Potential In Vitro," Proc. Natl. Acad. S: USA, Vol. 76, No. 3 pp. 1274-1278 (March 1979).

3.0 ml of the liquid medium was removed and replaced daily without disturbing the cell laden matrix. Glucose concentration was measured in the removed medium using a Sigma Diagnostic Glucose HK hexokinase enzymatic assay available from Sigma - Aldrich Co. in St. Louis, Missouri. Using standard ELISA assay techniques, the concentration of Protein C was also determined.

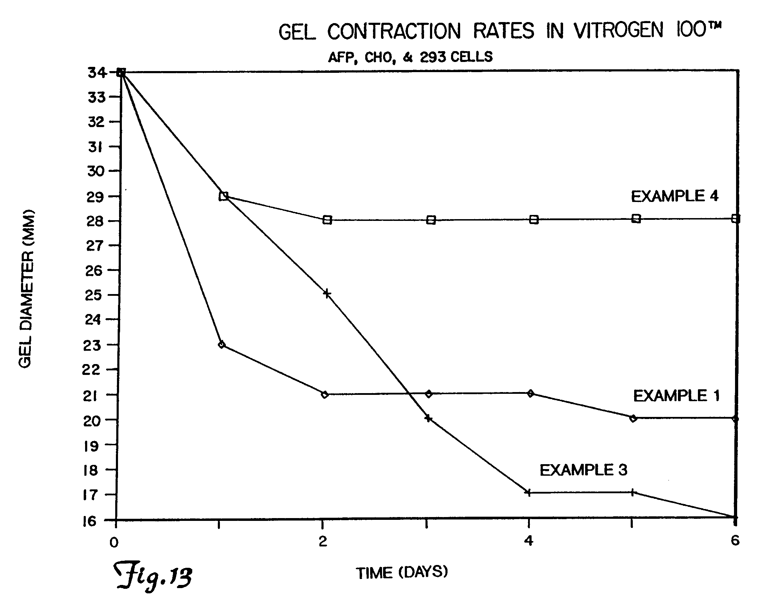

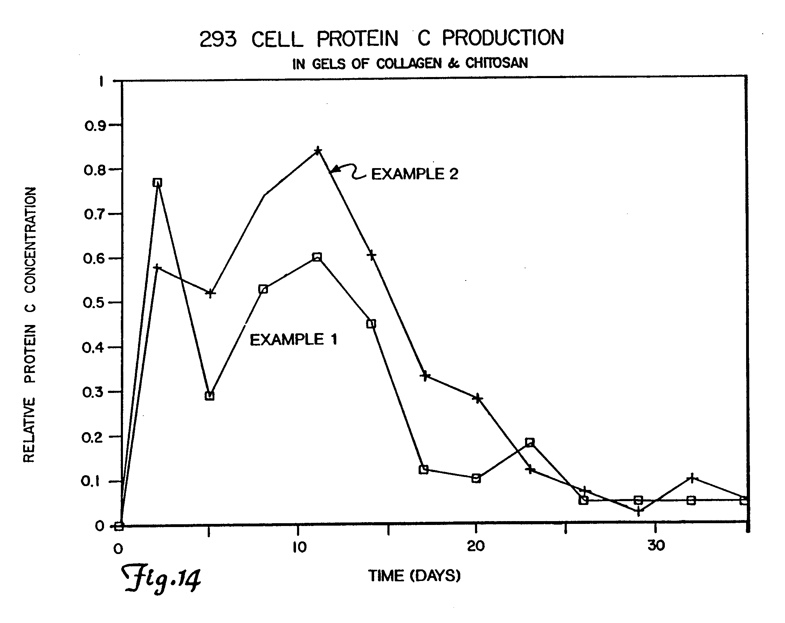

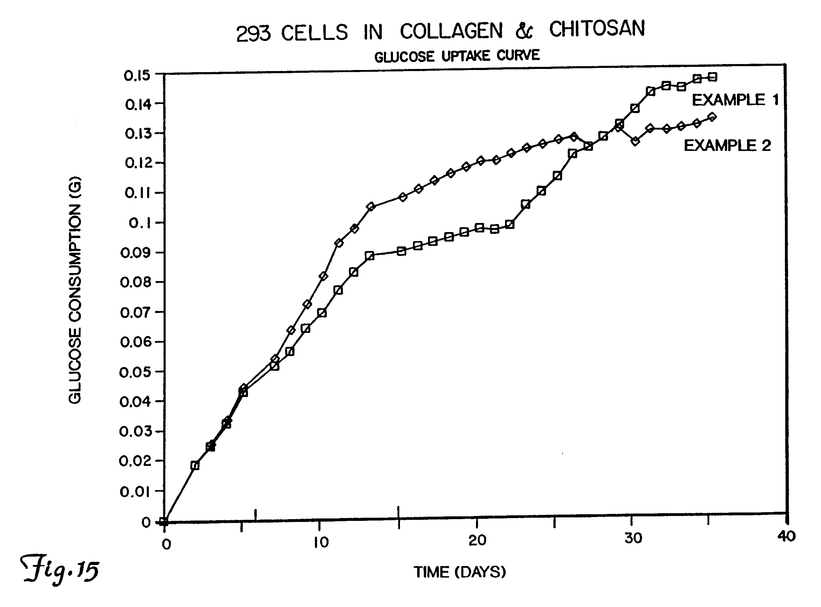

Figure 13 shows the rate of matrix contraction by comparing the gel diameter against time. After an initially high rate of contraction, the diameter of the cell matrix was generally stable. Figure 14 represents the concentration of Protein C that was contained in the spent medium. The glucose uptake curve of Figure 15 verifies the continued viability of the cells after being incorporated in the polymer matrix.

### EXAMPLE 2: 293 Cells in a Collagen - Chitosan Matrix

For this experiment the procedure of Example 1 was used except that Tube B further included 0.5 ml of a 2% aqueous solution of chitosan prepared by dissolving Ultrapure Chitosan (Protan Labs, Lot No. PTL-173) in distilled water, steam sterilized at 121°C for 30 minutes and then adjusted to pH 4. In the resulting solution the collagen concentration was reduced to 0.1% mg/ml. Using this mixture in Tube B a chitosan-collagen-cell matrix was created. Figure 14 demonstrates the successful production of Protein C over a prolonged period of time when the cells were incorporated in the biocompatible matrix. As shown in Figure 15, the cells continued to consume glucose while entrapped in this matrix.

### EXAMPLE 3: Chinese Hamster Ovary Cells in a Collagen Matrix

The protocol of Example 1 was modified to test the cell growth and contraction of Chinese Hamster Ovary Cells (CHO) in a collagen matrix. In this example Tube A held 1.05 ml. of a double concentration of DME, containing 10% by volume FBS; 200 units/ml penicillin G; 200 »g/ml streptomycin; and 0.06 ml. of 0.1 N Sodium hydroxide.

The CHO cells were prepared for use according to standard and well known techniques, e.g. V.B. Himes and W.S. Hu, Attachment and Growth of Mammalian Cells on Microcarriers with Different Ion Exchange Capacities, supra. The cells were subsequently suspended in a DME solution having 5% by volume FBS. 37.5 ml of a hamster cell suspension (7 x 10⁵ cells/ml) was centrifuged. Medium was removed until only 3 ml of medium remained, increasing the hamster cell concentration to 8.75 x 10⁶ cells/ml.

1.5 ml of the CHO cell suspension (1.31 x 10⁷ total cells) was added to the mixed contents of Tubes A and B. The mixture was then poured into a petridish as explained in Example 1. However, rather than incubating the petridish, the dish was floated on a 37°C water bath. In this way, the contents were rapidly warmed and fibrillogenesis of the collagen was forced to occur before the cells settled. After the substantially insoluble cell matrix formed, 5.0 ml of DME with 5% FBS and 100 units/ml Penicillin G and 100»g/ml Streptomycin was gently added to the surface of the cell matrix gel. Approximately 7.0 ml of medium was changed on a daily basis.

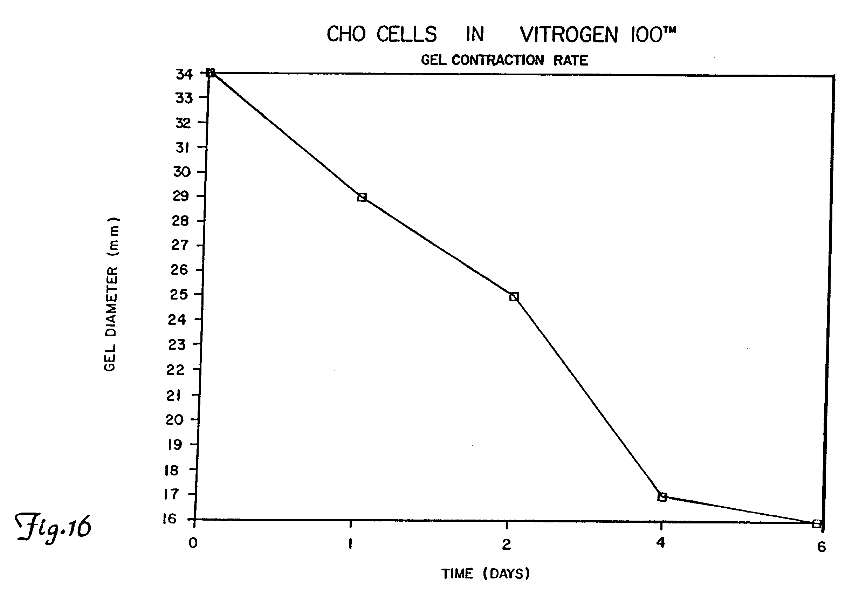

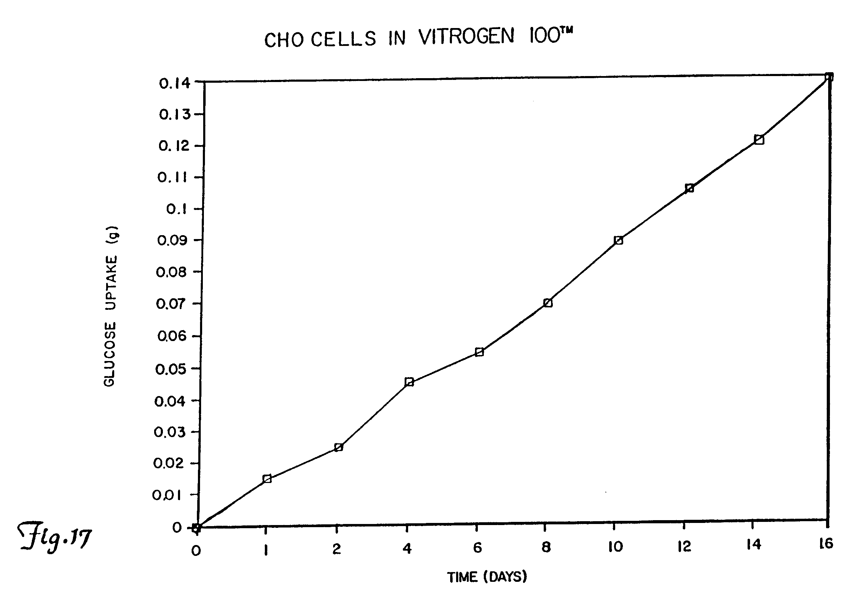

Figures 13 and 16 illustrate the rapid contraction of the cell-collagen mixture as the generally dense cell-collagen matrix was formed. The hamster cells also were successfully maintained in this biocompatible matrix as shown by the glucose uptake curve of Figure 17.

### EXAMPLE 4: AFP-27 Hybridoma Cells in a Collagen Matrix

Following the general protocol of Example 1, the following modifications were made to examine AFP-27 hybridoma cells ("AFP-27 cells") in a collagen matrix. AFP-27 cells produce IgG antibody to alpha fetal protein. These cells were obtained from Dr. Robert L. Vessella at the V.A. Medical Center in Minneapolis, Minnesota.

The solution of Tube A included 1 ml. of a double concentrated DME solution having 20% by volume horse serum; 200 units/ml. Penicillin G; 200 »g/ml Streptomycin; and 0.12 ml. of 0.1 N NaOH. Using the cell concentration technique set forth in Example 3, 30.8 ml of the AFP cell suspension (1.00 x 10⁶ cells/ml) was concentrated to 1.03 x 10⁷ cells/ml.

After Tube A and Tube B were mixed, 1.5 ml of the AFP cell suspension (1.54 x 10⁷ total cells) was added to the mixture. The total mixture was poured into a petridish and floated in a 37°C water bath as done in Example 3. After the collagen matrix formed, 4 ml of DME containing 10% horse serum, 100 units/ml Penicillin G and 100 »g/ml Streptomycin was added to the dish. Approximately 8.0 ml of medium was changed daily.

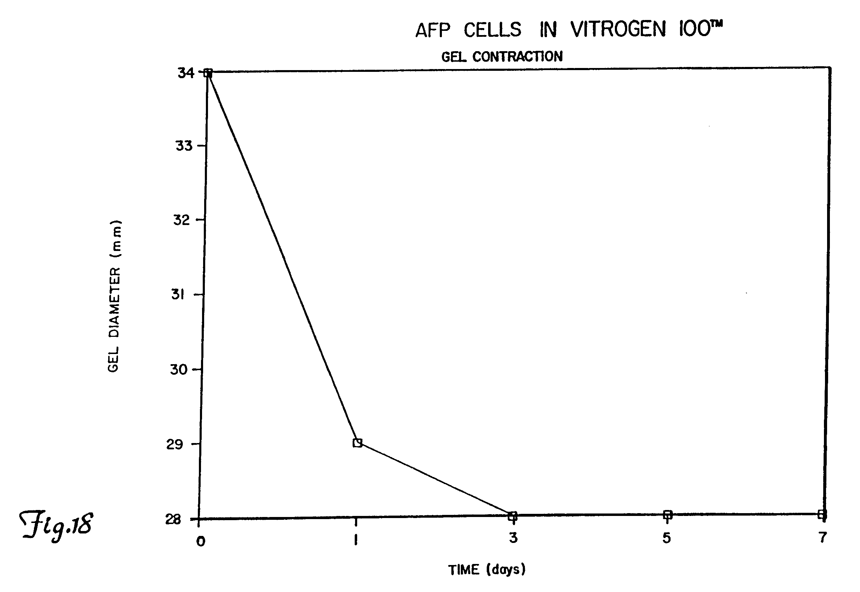

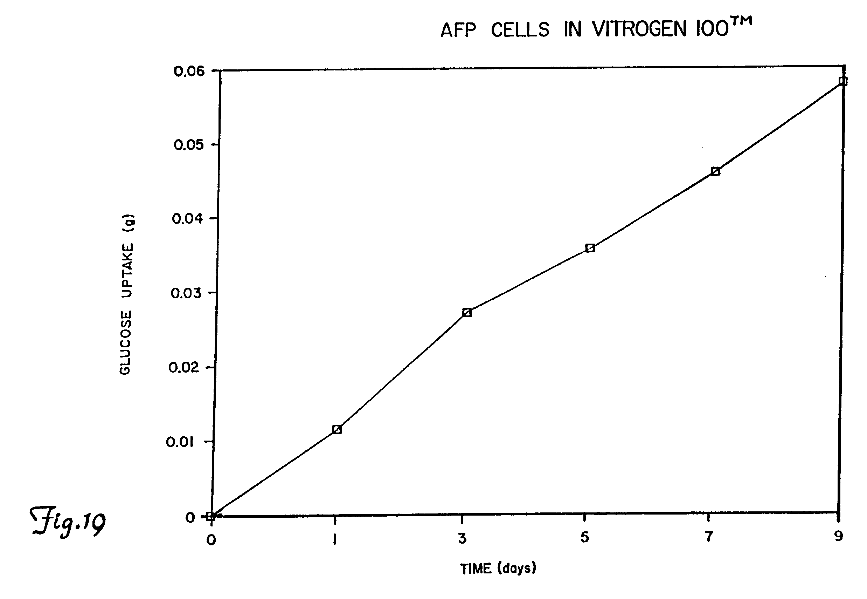

Figures 13 and 18 depict the formation of the substantially insoluble cell-collagen matrix over time. Figure 13 further compares the relative densities of the matrices formed in Examples 1, 3 and 4. The matrix of Example 3 was found to have the least diameter. The matrix of Example 4 had the largest diameter. Figure 19 demonstrates that this cell type can be maintained in this matrix environment over a sustained time period without loss of cell viability as evidenced by the continuous glucose uptake by the entrapped cells.

### EXAMPLE 5: 293 cells in a Fibrin Matrix

A solution of fibrinogen in a serum free medium was prepared by adding 0.075 g of bovine fibrinogen (Cat. No. F-4753 from Sigma Chemical Co. of St. Louis, Missouri) to 15.0 ml of a modified DME/F12 solution. This modified DME/F12 solution was made by mixing three parts of DME with 1 part Ham's F12 nutrient mixture (Gibco P.N. 430-1700), followed by addition of 300 »g/ml geneticin, 200 »g/ml hygromycin B and 1 »g/ml of Vitamin K (1 »g/ml). After the fibrinogen solution was mixed for 1 hour, the solution was decanted to remove any undissolved fibrinogen. The solution was then filter sterilized. A 1 unit/ml solution of thrombin was prepared by consecutive dilution of Thrombostat™ in PBS. Thrombostat™ is commercially available from Parke Davis in Morris Plains, New Jersey.

2.0 ml of the fibrinogen solution was added to Tube A. 0.2 ml of the thrombrin solution was added to Tube B. The tubes were sealed and chilled in ice water.

Using the cell suspension employed in Example 1, 0.9 ml of the cell suspension was added to Tube A and mixed. The contents of Tube B was then added to Tube A. The resulting mixture was immediately poured into a 34 mm diameter tissue culture petriplate. The plate was covered and incubated at 37°C for 30 minutes. After incubation, 3.0 ml of the DME/F12 solution was added to the petriplate. Using these techniques, a fibrin - cell matrix was successfully formed, entrapping most of the cells, although the matrix was subsequently degraded by fibrin degrading enzymes produced by the 293 cells. However, fibrin can still be used with a variety of cell types that do not produce similar hemolytic or degrading factors, such as AFP-27 hybridoma.

These examples demonstrate how a variety of cells can be incorporated and maintained in a biocompatible, substantially insoluble matrix. Using this matrix entrapping technique, the desired cell products can be harvested without disturbing the cells allowing for a continued high concentration of cell product. The substantially insoluble matrix also allows for the continuous secretion of cell product over time without interfering with cell viability. The following example uses a matrix formed in situ in a flat bed embodiment of a bioreactor apparatus of the present invention.

### EXAMPLE 6: 293 Cells in a Collagen Supported Bioreactor Apparatus

Using flat bed reactor 100, which had been previously assembled and steam sterilized the following experiment was performed, again using most of the techniques described more fully in Examples 1 through 5. The contents of Tube A included 7.2 ml of twice concentrated DME solution, 10% by volume FBS and 0.48 ml 0.1 N NaOH. Tube B held 5.4 ml of VITROGEN 100™.

293 cells were trypsinized as discussed in Example 1. The resulting cell suspension had a concentration of 5.20 x 10⁶ cells/ml. Using aseptic techniques, the contents of Tube A was added to Tube B, and mixed well. Immediately thereafter, the cell suspension was added to Tube B to form the matrix precursor-cell suspension. The matrix precursor-cell suspension was then quickly injected through second fluid inlet means 132 and into cell growth plate window(s) 144.

Medium reservoir 156 was filled with 300 ml of DME containing 5% by volume FBS, 300 »g/ml geneticin, 200 »g/ml hydrogromycin B and 1 »g/ml Vitamin K. Medium was pumped from reservoir 156 through bioreactor 100. The whole apparatus was placed in a room having a temperature of approximately 37°C. Samples were taken daily from cell growth plate window 144 through a "T" valve in flow communication with second fluid outlet means 136, in order to analyze pH, glucose and cell product concentration. Cells have been maintained successfully in this apparatus for 90 days with a continual production of Protein C.

### Example 7: 293 Cells in Collagen Supported Hollow Fiber Reactor

Using the hollow fiber bioreactor apparatus 200, the following experiment was conducted, again using the techniques described in Examples 1 through 5. To sterile Tube A a 7.0 ml solution was added consisting of twice concentrated DME, containing 10% by volume FBS, 600 »g/ml geneticin, 400 »g/ml hygromycin B, 2 »g/ml Vitamin K plus 0.4 ml of 0.1 N NaOH. Tube B contained 7.0 ml of VITROGEN 100™. The tubes were then placed in an ice water bath.

Hollow fiber assembly 200 was flushed with 5 l. of distilled water and sterilized by immersion in distilled water with steam sterilization for 30 minutes at approximately 121°C. Reservoir 212, and all other units of the reactor were also steam sterilized. Following sterilization, the entire assembly was cooled to 4°C. and assembled aseptically in a laminar flow hood.

293 cells were trypsinized as discussed in Example 1, resulting with 5.25 ml of a cell suspension having a concentration of 1.47 x 10⁷ cells/ml. Using aseptic techniques, the contents of Tube A were added to Tube B and mixed well. The resulting mixture was then immediately combined with the 293 cell suspension to form the cell-matrix precursor mixture. This cell-matrix precursor mixture was introduced into hollow fibers 210 through first fluid inlet means 202.

Reservoir 212 was filled with 300 ml of DME containing 5% FBS, 300 »g/ml geneticin 200 »g/ml hygromycin B and 1»g/ml Vitamin K.
Hydroxyethylpiperazine ethylsulfonic acid (HEPES) (8 g/l) was also added to the medium reservoir in place of sodium bicarbonate. Medium was pumped from reservoir 212 through second fluid inlet means 204, extracapillary space 216 and second fluid outlet means 208. Small samples were taken daily from first fluid inlet means 202 and analyzed for pH, glucose and Protein C concentration. Small aliquots of 1N NaOH were added periodically to maintain the pH in the range of 7.0 - 7.4. Using this system, the cells were successfully maintained for 50 days. Cell product was continually collected over this time period.

While many specific embodiments have been shown and described in detail to illustrate the application of the principles of this invention, it will be understood by those skilled in the art that the invention may be embodied otherwise without departing from such principles. For example, while the hollow fiber assembly was described using a conventional nutrient medium flow traveling along the length of the hollow fibers in the extracapillary space, a crossflow system may also be used such that nutrient medium would flow generally perpendicular to the hollow fibers. Indeed, a crossflow system may provide a higher oxygen transfer to a greater proportion of entrapped cells.

## Claims

1. A method for maintaining cells in vitro over a sustained period of time for continuous production of a desired cell product, said cells being selected from the group consisting of animal cells, genetically altered animal cells and mixtures thereof, said method comprising the steps of:
(a) introducing a matrix precursor including said cells into a cell chamber;
(b) inducing formation of an at least partially insoluble cell-biocompatible matrix means;
(c) contracting said at least partially insoluble cell-biocompatible matrix means including said cells in situ within said cell chamber, to form a contracted at least partially insoluble phase for entrapping said cells and a liquid phase for accumulating the desired cell product;
(d) supplying nutrient medium for said cells by passing said nutrient medium through an inlet means leading to a chamber for cell nutrient and cell waste, said chamber for cell nutrient and cell waste separated from said cell chamber by a selectively permeable membrane, and perfusing said nutrient medium through said membrane into said cell chamber; and
(e) withdrawing expended nutrient medium and cell waste which have travelled from said cell chamber to said chamber for cell nutrient and cell waste through said permeable membrane from the chamber for cell nutrient and cell waste through an outlet means.

2. The method of claim 1, further comprising the step of withdrawing the desired cell product from said cell chamber through a second outlet means in flow communication with said cell chamber.

3. The method of claim 1 or 2, wherein said matrix means is formed from teleopeptide native collagen, ateleopeptide native collagen or modified collagen.

4. The method of claim 1 or 2, wherein said matrix means is formed from a collagen-chitosan mixture.

5. The method of claim 1 or 2, wherein said matrix means is formed from fibrin.

6. The method according to one of the claims 1 to 5, wherein said membrane is a processed cellulose derivative which is permeable to low molecular weight nutrient and cell waste product compounds, substantially impermeable to high molecular weight desired cell product compounds, and has an upper molecular weight permeability limit equal to or less than that of the desired cell product.

7. The method according to one of the claims 1 to 5, wherein said membrane is one of polysulfone, Teflon® and ceramic.

8. The method according to one of the claims 1 to 7, wherein said selectively permeable membrane allows the passage of compounds having a molecular weight below about 12,000.

9. The method according to one of the claims 1 to 7, wherein said selectively permeable membrane allows the passage of compounds having a molecular weight below about 30,000.

10. The method according to one of the claims 1 to 7, wherein said selectively permeable membrane allows the passage of compounds having a molecular weight below about 100,000.

11. The method according to one of the claims 1 to 10, wherein said contracting step comprises contracting said matrix means to approximately 75% of the original volume occupied within said cell chamber by said matrix precursor and said cells.

12. The method according to one of the claims 1 to 11, wherein said cells are Chinese hamster ovary cells.

13. The method of claim 12, further comprising introducing serum free medium as said nutrient medium into said chamber for cell nutrient and cell waste such that cell viability is maintained as is determinable through detection of no substantial decrease in glucose consumption rate of said cells.

14. The method according to one of the claims 1 to 11, wherein said cells are hybridoma cells.

15. The method of claim 12, further comprising adding protein free cell culture medium as said medium into said chamber for cell nutrient and cell waste.

16. A bioreactor apparatus for maintaining cells in vitro over a sustained period of time for continuous or periodic production of a desired cell product, said cells being selected from the group of animal cells, genetically altered animal cells and mixtures thereof, said apparatus comprising:
(a) a housing having a proximal end and a distal end;
(b) at least one selectively permeable membrane within said housing extending from said proximal to said distal end of said housing, said membrane dividing the interior of said housing into a cell chamber and a chamber for cell nutrient and cell waste, said membrane selectively allowing cell nutrient and cell waste to cross between said chambers but retaining the desired cell product within the cell chamber;
(c) said cell chamber being subdivided into two phases comprising a liquid zone for accumulating the desired cell product, and a substantially insoluble, biocompatible matrix, the matrix comprising a fibrous gel formed in situ from a solution of matrix precursors in the cell chamber, and cells being entrapped within the gel during the formation thereof; and
(d) means communicating with said chamber for cell nutrient and cell waste for supplying nutrient medium for maintaining said cells and for withdrawing expended cell nutrient and cell waste.

17. The apparatus of claim 16, further comprising means in flow communication with said cell chamber for withdrawing the desired cell product.

18. The apparatus according to claim 16 or 17, further comprising means in flow communication with said cell chamber for introducing a matix precursor-cell suspension into said cell chamber.

19. The apparatus according to one of the claims 16 to 18, further comprising a second selectively permeable membrane extending from said proximal to said distal end of said housing forming a second chamber for cell nutrient and cell waste with said cell chamber intermediate to said first and second chambers for cell nutrient and cell waste.

20. The apparatus of claim 19, further comprising a plurality of selectively permeable membranes extending from said proximal to said distal end of said housing forming a plurality of said chambers for cell nutrient and cell waste and a plurality of said cell chambers such that said chambers for cell nutrient and cell waste and said cell chambers alternate within said housing.

21. The apparatus according to one of the claims 16 to 20, wherein said biocompatible matrix has contracted in situ to less than about 90% of the original volume occupied by the matrix precursor-cell suspension within said cell chamber.

22. The apparatus according to one of the claims 16 to 20, wherein said biocompatible matrix has contracted in situ to less than about 75% of the original volume occupied by the matrix precursor-cell suspension within said cell chamber.

23. The apparatus according to one of the claims 16 to 20, wherein said biocompatible matrix has contracted in situ to less than about 50% of the original volume occupied within said cell chamber by the matrix precursor-cell suspension.

24. The apparatus according to one of the claims 16 to 20, wherein said biocompatible matrix has contracted in situ to less than about one-third of the original volume occupied within said cell chamber by the matrix precursor-cell suspension.

25. The apparatus according to one of the claims 16 to 24, wherein said membrane is composed of a cellulose derivative which is permeable to low molecular weight nutrient and cell waste product compounds, substantially impermeable to high molecular weight desired cell product compounds, and has an upper molecular weight permeability limit equal to or less than that of the desired cell product.

26. The apparatus according to one of the claims 16 to 24, whrein said membrane is constructed of a material selected from the group consisting of polysulfone, polytetrafluoroethylene and ceramic.

27. The apparatus according to one of the claims 16 to 26, wherein said biocompatible matrix is formed from teleopeptide native collagen or ateleopeptide native collagen or modified collagen.

28. The apparatus according to one of the claims 16 to 26, wherein said biocompatible matrix is formed from chitosan and modified chitosan.

29. The apparatus according to one of the claims 16 to 26, wherein said biocompatible matrix is formed from a collagen-chitosan mixture.

30. The apparatus according to one of the claims 16 to 26, wherein said biocompatible matrix is formed from fibrin.

31. The apparatus according to one of the claims 16 to 26, wherein said biocompatible matrix is formed from a polyamine which is soluble in cell culture medium at pH values ranging from 2 to 5.5 and is at least partially insoluble in cell culture medium at pH values ranging from 6.8 to 7.4.

32. The apparatus according to one of the claims 16 to 26, wherein said biocompatible matrix is fromed from a mixture of polyanionic polymers and polycationic polymers.

33. The apparatus according to one of the claims 16 to 26, wherein said biocompatible matrix is formed from a polymer that is soluble in cell culture medium at temperatures ranging from 0°C to 30°C and is at least partially insoluble in cell culture medium at temperatures ranging from 32°C to 45°C.

## Patentansprüche

1. Verfahren zum Aufrechterhalten von Zellen in vitro über eine ununterbrochene Zeitdauer zur kontinuierlichen Herstellung eines gewünschten Zellproduktes, wobei die Zellen aus der Gruppe ausgewählt sind, die aus Tierzellen, genetisch veränderten Tierzellen und Gemischen davon besteht, mit den folgenden Schritten:
(a) Einführen eines Matrix-Vorläufers, der die Zellen aufweist, in eine Zellkammer;
(b) Induzieren der Ausbildung einer zumindest teilweise unlöslichen zell-biocompatiblen Matrix-Einrichtung;
(c) Kontrahieren der mindestens teilweise unlöslichen zell-biocompatiblen Matrix-Einrichtung einschließlich der Zellen in situ innerhalb der Zellkammer, um eine kontrahierte, zumindest teilweise unlösliche Phase zum Einfangen der Zellen und eine flüssige Phase zum Ansammeln des gewünschten Zellproduktes zu bilden;
(b) Zuführen eines Nährmediums für die Zellen durch Führen des Nährmediums durch eine Einlaßeinrichtung, die zu einer Kammer für Zellnährstoff und Zellabfall führt, wobei diese Kammer für Zellnährstoff und Zellabfall durch eine selektiv permeable Membran von der Zellkammer getrennt ist, und Führen des Nährmediums durch die Membran in die Zellkammer; und
(e) Abziehen des verbrauchten Nährmediums und Zellabfalls, die sich von der Zellkammer zur Kammer für Zellnährstoff und Zellabfall durch die permeable Membran bewegt haben, von der Kammer für Zellnährstoff und Zellabfall durch eine Auslaßeinrichtung.

2. Verfahren nach Anspruch 1, das des weiteren den Schritt des Abziehens des gewünschten Zellproduktes aus der Zellkammer durch eine zweite Auslaßeinrichtung, die in Strömungsmittelverbindung mit der Zellkammer steht, umfaßt.

3. Verfahren nach Anspruch 1 oder 2, bei dem die Matrix-Einrichtung aus Teleopeptid-Nativ-Kollagen, Ateleopeptid-Nativ-Kollagen oder modifiziertem Kollagen gebildet wird.

4. Verfahren nach Anspruch 1 oder 2, bei dem die Matrix-Einrichtung aus einem Kollagen-Chitosan-Gemisch gebildet wird.

5. Verfahren nach Anspruch 1 oder 2, bei dem die Matrix-Einrichtung aus Fibrin gebildet wird.

6. Verfahren nach einem der Ansprüche 1 bis 5, bei dem die Membran ein behandeltes Cellulosederivat ist, das gegenüber Nährstoff- und Zellabfallproduktverbindungen mit niedrigem Molekulargewicht durchlässig ist, gegenüber den gewünschten Zellproduktverbindungen mit hohem Molekulargewicht im wesentlichen undurchlässig ist und eine obere Molekulargewichtspermeabilitätsgrenze aufweist, die der des gewünschten Zellproduktes entspricht oder geringer als diese ist.

7. Verfahren nach einem der Ansprüche 1 bis 5, bei dem die Membran aus Polysulfon, Teflon und Keramik ausgewählt ist.

8. Verfahren nach einem der Ansprüche 1 bis 7, bei dem die selektiv permeable Membran den Durchgang von Verbindungen ermöglicht, die ein Molekulargewicht unter etwa 12.000 besitzen.

9. Verfahren nach einem der Ansprüche 1 bis 7, bei dem die selektiv permeable Membran den Durchgang von Verbindungen ermöglicht, die ein Molekulargewicht unter etwa 30.000 besitzen.

10. Verfahren nach einem der Ansprüche 1 bis 7, bei dem die selektiv permeable Membran den Durchgang von Verbindungen ermöglicht, die ein Molekulargewicht unter etwa 100.000 besitzen.

11. Verfahren nach einem der Ansprüche 1 bis 10, bei dem der Kontrahierungsschritt das Kontrahieren der Matrix-Einrichtung auf etwa 75 % des ursprünglichen Volumens, das vom Matrix-Vorläufer und den Zellen in der Zellkammer eingenommen wurde, umfaßt.

12. Verfahren nach einem der Ansprüche 1 bis 11, bei dem die Zellen Eierstockzellen des chinesischen Hamsters sind.

13. Verfahren nach Anspruch 12, das des weiteren das Einführen von serumfreiem Medium als Nährmedium in die Kammer für den Zellnährstoff und Zellabfall umfaßt, so daß die Lebensfähigkeit der Zelle aufrechterhalten wird, wie dies durch Detektion von keinem wesentlichen Abfall des Glucose-Verbrauchs der Zellen feststellbar ist.

14. Verfahren nach einem der Ansprüche 1 bis 11, bei dem die Zellen Hybridoma-Zellen sind.

15. Verfahren nach Anspruch 12, das des weiteren das Zusetzen eines proteinfreien Zellkulturmediums als Medium in die Kammer für den Zellnährstoff und den Zellabfall umfaßt.

16. Bioreaktor zur Aufrechterhaltung von Zellen in vitro über eine ununterbrochene Zeitdauer zur kontinuierlichen oder periodischen Herstellung eines gewünschten Zellproduktes, wobei die Zellen aus der aus Tierzellen, genetisch veränderten Tierzellen und Gemischen davon bestehenden Gruppe ausgewählt sind, mit:
(a) einem Gehäuse mit einem proximalen Ende und einem distalen Ende;
(b) mindestens einer selektiv permeablen Membran innerhalb des Gehäuses, die sich vom proximalen Ende bis zum distalen Ende des Gehäuses erstreckt, das Innere des Gehäuses in eine Zellkammer und eine Kammer für Zellnährstoff und Zellabfall unterteilt und wahlweise einen Austausch des Zellnährstoffs und Zellabfalls zwischen den Kammern ermöglicht, jedoch das gewünschte Zellprodukt innerhalb der Zellkammer zurückbehält;
(c) wobei die Zellkammer in zwei Phasen unterteilt ist, die eine flüssige Zone zum Ansammeln des gewünschten Zellproduktes und eine im wesentlichen unlösliche, biocompatible Matrix umfassen, welche ein vibröses Gel, das in situ aus einer Lösung der Matrix-Vorläufer in der Zellkammer gebildet worden ist, und Zellen umfaßt, die während ihrer Bildung im Gel eingefangen worden sind; und
(d) Einrichtungen, die mit der Kammer für den Zellnährstoff und den Zellabfall in Verbindung stehen und Nährmedium zum Aufrechterhalten der Zellen zuführen sowie verbrauchten Zellnährstoff und Zellabfall abziehen.

17. Vorrichtung nach Anspruch 16, die des weiteren eine Einrichtung aufweist, die in Strömungsmittelverbindung mit der Zellkammer steht, um das gewünschte Zellprodukt abzuziehen.

18. Vorrichtung nach Anspruch 16 oder 17, die des weiteren eine Einrichtung aufweist, die in Strömungsmittelverbindung mit der Zellkammer steht, um eine Matrix-Vorläufer-Zellsuspension in die Zellkammer einzuführen.

19. Vorrichtung nach einem der Ansprüche 16 bis 18, die des weiteren eine zweite selektiv permeable Membran umfaßt, die sich vom proximalen zum distalen Ende des Gehäuses erstreckt und eine zweite Kammer für Zellnährstoff und Zellabfall bildet, wobei die Zellkammer zwischen der ersten und zweiten Kammer für Zellnährstoff und Zellabfall angeordnet ist.

20. Vorrichtung nach Anspruch 19, die des weiteren eine Vielzahl von selektiv permeablen Membranen aufweist, die sich vom proximalen zum distalen Ende des Gehäuses erstrecken und eine Vielzahl von Kammern für Zellnährstoff und Zellabfall und eine Vielzahl von Zellkammern bilden, so daß die Kammern für Zellnährstoff und Zellabfall und die Zellkammern abwechselnd im Gehäuse angeordnet sind.

21. Vorrichtung nach einem der Ansprüche 16 bis 20, bei der die biocompatible Matrix in situ auf weniger als etwa 90 % des von der Matrix-Vorläufer-Zellsuspension in der Zellkammer eingenommenen ursprünglichen Volumens kontrahiert wurde.

22. Vorrichtung nach einem der Ansprüche 16 bis 20, bei der die biocompatible Matrix in situ auf weniger als etwa 75 % des von der Matrix-Vorläufer-Zellsuspension innerhalb der Zellkammer eingenommenen Volumens kontrahiert wurde.

23. Vorrichtung nach einem der Ansprüche 16 bis 20, bei der die biocompatible Matrix in situ auf weniger als etwa 50 % des von der Matrix-Vorläufer-Zellsuspension innerhalb der Zellkammer eingenommenen ursprünglichen Volumens kontrahiert wurde.

24. Vorrichtung nach einem der Ansprüche 16 bis 20, bei der die biocompatible Matrix in situ auf weniger als etwa 1/3 des von der Matrix-Vorläufer-Zellsuspension innerhalb der Zellkammer eingenommenen ursprünglichen Volumens kontrahiert wurde.

25. Vorrichtung nach einem der Ansprüche 16 bis 24, bei der die Membran aus einem Cellulose-Derivat besteht, das gegenüber Nährstoff- und Zellabfallproduktverbindungen mit niedrigem Molekulargewicht durchlässig ist, gegenüber den gewünschten Zellproduktverbindungen mit hohem Molekulargewicht im wesentlichen undurchlässig ist und eine obere Molekulargewichtsdurchlässigkeitsgrenze aufweist, die der des gewünschten Zellproduktes entspricht oder geringer als diese ist.

26. Vorrichtung nach einem der Ansprüche 16 bis 24, bei der die Membran aus einem Material konstruiert ist, das aus der Gruppe ausgewählt ist, die aus Polysulfon, Polytetrafluorethylen und Keramik besteht.

27. Vorrichtung nach einem der Ansprüche 16 bis 26, bei der die biocompatible Matrix aus Teleopeptid-Nativ-Kollagen oder Ateleopeptide-Nativ-Kollagen oder modifiziertem Kollagen gebildet ist.

28. Vorrichtung nach einem der Ansprüche 16 bis 26, bei der die biocompatible Matrix aus Chitosan und modifiziertem Chitosan gebildet ist.

29. Vorrichtung nach einem der Ansprüche 16 bis 26, bei der die biocompatible Matrix aus einem Kollagen-Chitosan-Gemisch gebildet ist.

30. Vorrichtung nach einem der Ansprüche 16 bis 26, bei der die biocompatible Matrix aus Fibrin gebildet ist.

31. Vorrichtung nach einem der Ansprüche 16 bis 26, bei der die biocompatible Matrix aus einem Polyamin gebildet ist, das in Zellkulturmedium bei pH-Werten von 2 bis 5,5 löslich und in Zellkulturmedium bei pH-Werten von 6,8 bis 7,4 zumindest teilweise unlöslich ist.

32. Vorrichtung nach einem der Ansprüche 16 bis 26, bei der die biocompatible Matrix aus einem Gemisch aus polyanionischen Polymeren und polykationischen Polymeren gebildet ist.

33. Vorrichtung nach einem der Ansprüche 16 bis 26, bei der die biocompatible Matrix aus einem Polymer gebildet ist, das in Zellkulturmedium bei Temperaturen von 0°C bis 30°C löslich und in Zellkulturmedium bei Temperaturen von 32°C bis 45°C teilweise unlöslich ist.

## Revendications

1. Procédé pour maintenir des cellules in vitro pendant une période de temps soutenu pour la production continue d'un produit cellulaire souhaité, lesdites cellules étant sélectionnées à partir d'un groupe constitué de cellules animales, des cellules animales génétiquement modifiées et des mélanges de celles-ci, ledit procédé comprenant les étapes de :
a) introduire un précurseur de matrice comprenant lesdites cellules dans une chambre cellulaire ;
b) provoquer la formation de moyens de matrice cellulairement bio-compatible au moins partiellement insolubles ;
c) contracter lesdits moyens de matrice cellulairement bio-compatibles au moins partiellement insolubles contenant lesdites cellules in situ à l'intérieur de ladite chambre cellulaire pour former une phase contractée au moins partiellement insolubles pour piéger lesdites cellules et une phase liquide pour accumuler le produit cellulaire souhaité ;
d) fournir un milieu nutritif pour lesdites cellules en passant ledit milieu nutritif à travers des moyens d'entrée conduisant à une chambre pour nutriments cellulaires et déchets cellulaires, ladite chambre pour nutriments cellulaires et déchets cellulaires étant séparée de ladite chambre cellulaire par une membrane sélectivement perméable, et perfuser ledit milieu nutritif à travers ladite membrane à l'intérieur de ladite chambre cellulaire ; et
e) retirer le milieu nutritif consommé et les déchets cellulaires qui se sont déplacés à partir de ladite chambre cellulaire vers ladite chambre pour nutriments cellulaires et déchets cellulaires à travers ladite membrane perméable à partir de la chambre pour nutriments cellulaires et déchets cellulaires à travers des moyens de sortie.

2. Procédé selon la revendication 1, comprenant en outre l'étape de retirer le produit cellulaire souhaité de ladite chambre cellulaire à travers des seconds moyens de sortie en communication d'écoulement avec ladite chambre cellulaire.

3. Procédé selon la revendication 1 ou la revendication 2, dans lequel lesdits moyens de matrice sont formés à partir de collagènes naturels téléopeptides, de collagènes naturels atéléopeptides ou de collagènes modifiés.

4. Procédé selon la revendication 1 ou la revendication 2, dans lequel lesdits moyens de matrice sont formés à partir d'un mélange collagène chitosan.

5. Procédé selon la revendication 1 ou la revendication 2, dans lequel ledits moyens de matrice sont formés à partir de fibrine.

6. Procédé selon l'une quelconque des revendications 1 à 5, dans lequel ladite membrane est un dérivé de cellulose traité qui est perméable aux composants de produits de nutriments et de déchets cellulaires de faible poids moléculaires, sensiblement imperméables aux composants de produits cellulaires souhaités de poids moléculaires élevé, et a une limite supérieure de perméabilité au poids moléculaire égal ou inférieur à celle du produit cellulaire souhaité.

7. Procédé selon l'une quelconque des revendications 1 à 5, dans lequel ladite membrane est choisie parmi le polysulfone, le Teflon® et la céramique.

8. Procédé selon l'une des revendication 1 à 7, dans lequel ladite membrane sélectivement perméable permet le passage de composants ayant un poids moléculaire inférieur à environ 12 000.

9. Procédé selon l'une des revendications 1 à 7, dans lequel ladite membrane sélectivement perméable permet le passage de composants ayant un poids moléculaire inférieur à environ 30 000.

10. Procédé selon l'une des revendications 1 à 7, dans lequel ladite membrane sélectivement perméable permet le passage de composants ayant un poids moléculaire inférieur à environ 100 000.

11. Procédé selon l'une des revendications 1 à 10, dans lequel ladite étape de contraction comprend de contracter lesdits moyens de matrice à environ 75% du volume d'origine occupée à l'intérieur de ladite chambre cellulaire par ledit précurseur de matrice et lesdites cellules.

12. Procédé selon l'une des revendications 1 à 11, dans lequel lesdites cellules sont des cellules ovariennes de hamster chinois.

13. Procédé selon la revendication 12, comprenant en outre d'introduire un milieu sans sérum en tant que dit milieu nutritif à l'intérieur de ladite chambre pour nutriments cellulaires et déchets cellulaires, de telle sorte que la viabilité cellulaire est maintenue comme cela est déterminable par la détection d'aucune diminution substantielle du taux de consommation de glucose desdites cellules.

14. Procédé selon l'une des revendications 1 à 11, dans lequel lesdites cellules sont des cellules d'hybridomes.

15. Procédé selon la revendication 12, comprenant en outre d'ajouter un milieu de cultures cellulaires sans protéine en tant que dit milieu à l'intérieur de ladite chambre pour nutriments cellulaires et déchets cellulaires.

16. Appareil de bio-réacteur pour maintenir des cellules in vitro pendant une période de temps soutenue pour la production continue ou périodique d'un produit cellulaire souhaité, lesdites cellules étant sélectionnées à partir du groupe constitué des cellules animales, des cellules animales génétiquement modifiées et des mélanges de celles-ci, ledit appareil comprenant :
a) un boîtier ayant une extrémité proximale et une extrémité distale;
b) au moins une membrane sélectivement perméable à l'intérieur dudit boîtier s'étendant à partir de ladite extrémité proximale vers ladite extrémité distale dudit boîtier, ladite membrane divisant l'intérieur dudit boîtier en une chambre cellulaire et une chambre pour nutriments cellulaires et déchets cellulaires, ladite membrane permettant sélectivement aux nutriments cellulaires et aux déchets cellulaires de passer entre lesdites chambres tout en retenant le produit cellulaire souhaité à l'intérieur de la chambre cellulaire ;
c) ladite chambre cellulaire divisée en deux phases comprenant une zone liquide pour accumuler le produit cellulaire souhaité, et une matrice bio-compatible sensiblement insoluble, la matrice comprenant un gel fibreux formé in situ à partir d'une solution de précurseur de matrice dans la chambre cellulaire, et les cellules étant piégées dans le gel pendant la formation de celui-ci ; et
d) des moyens communiquant avec ladite chambre pour nutriments cellulaires et déchets cellulaires pour alimenter un milieu nutritif pour maintenir lesdites cellules et pour retirer les nutriments cellulaires consommés et les déchets cellulaires.

17. Appareil selon la revendication 16, comprenant en outre des moyens en communication d'écoulement avec ladite chambre cellulaire pour retirer le produit cellulaire souhaité.

18. Appareil selon la revendication 16 ou la revendication 17, comprenant en outre des moyens en communication d'écoulement avec ladite chambre cellulaire pour introduire une suspension de cellules-précurseur de matrice à l'intérieur de ladite chambre cellulaire.

19. Appareil selon les revendications 16 à 18, comprenant en outre une seconde membrane sélectivement perméable s'étendant à partir de ladite extrémité proximale vers ladite extrémité distale dudit boîtier formant d'une seconde chambre pour nutriments cellulaires et déchets cellulaires avec ladite chambre cellulaire située entre ladite première et seconde chambres pour nutriments cellulaires et déchets cellulaires.

20. Appareil selon la revendication 19, comprenant en outre une pluralité de membranes sélectivement perméables s'étendant à partir de ladite extrémité proximale vers ladite extrémité distale dudit boîtier formant une pluralité desdites chambres pour nutriments cellulaires et déchets cellulaires et une pluralité desdites chambres cellulaires de telle sorte que lesdites chambres pour nutriments cellulaires et déchets cellulaires et lesdites chambres cellulaires s'alternent à l'intérieur dudit boîtier.

21. Appareil selon l'une des revendications 16 à 20, dans lequel ladite matrice bio-compatibles est contractée in situ à moins d'environ 90% du volume d'origine occupée par la suspension de cellules-précurseur de matrice à l'intérieur de ladite chambre cellulaire.

22. Appareil selon l'une des revendications 16 à 20, dans lequel ladite matrice bio-compatibles est contractée in situ à moins d'environ 75% du volume d'origine occupée par la suspension de cellules-précurseur de matrice à l'intérieur de ladite chambre cellulaire.

23. Appareil selon l'une des revendications 16 à 20, dans lequel ladite matrice bio-compatibles est contractée in situ à moins d'environ 50% du volume d'origine occupée par la suspension de cellules-précurseur de matrice à l'intérieur de ladite chambre cellulaire.

24. Appareil selon l'une des revendications 16 à 20, dans lequel ladite matrice bio-compatibles est contractée in situ à moins d'environ un tiers du volume d'origine occupée par la suspension de cellules-précurseur de matrice à l'intérieur de ladite chambre cellulaire.

25. Appareil selon l'une des revendications 16 à 24, dans lequel ladite membrane est composée d'un dérivé de cellulose qui est perméable aux composés de produits de nutriments et de déchets cellulaires de faible poids moléculaire, sensiblement imperméable aux composés de produits cellulaires souhaités de poids moléculaire élevé et a une limite de perméabilité supérieure au poids moléculaire égale ou inférieure à celle du produit cellulaire souhaité.

26. Appareil selon l'une des revendications 16 à 24, dans lequel ladite membrane est constituée d'un matériau choisi dans le groupe constitué de la polysulfone, du polytetrafluoroéthylène et de la céramique.

27. Appareil selon l'une des revendications 16 à 26, dans lequel ladite matrice bio-compatible est formée à partir de collagènes naturels téléopeptides ou de collagènes naturels atéléopeperides ou de collagènes modifiés.

28. Appareil selon l'une des revendications 16 à 26, dans lequel ladite matrice bio-compatible est formée à partir de chitosans et de chitosans modifiés.

29. Appareil selon l'une des revendications 16 à 26, dans lequel ladite matrice bio-compatible est formée à partir d'un mélange collagène chitosan.

30. Appareil selon l'une des revendications 16 à 26, dans lequel ladite matrice bio-compatible est formée à partir de fibrine.

31. Appareil selon l'une des revendications 16 à 26, dans lequel ladite matrice bio-compatible est formée à partir d'une polyamine qui est soluble en milieu de cultures cellulaires ayant des valeurs de pH allant de 2 à 5,5 et est au moins partiellement insoluble en milieu de cultures cellulaires ayant des valeurs de pH allant de 6,8 à 7,4.

32. Appareil selon l'une des revendications 16 à 26, dans lequel ladite matrice bio-compatible est formée à partir d'un mélange de polymères polyanioniques et de polymères polycationiques.

33. Appareil selon l'une des revendications 16 à 26, dans lequel ladite matrice bio-compatible est formée à partir d'un polymère qui est soluble en milieu de cultures cellulaires à des températures allant de 0°C à 30°C et au moins partiellement insolubles en milieu de cultures cellulaires à des températures allant de 32°C à 45°C.
